# EUROPEAN PATENT SPECIFICATION

(11) **EP 2 711 358 B1**
(45) Date of publication and mention of the grant of the patent: **08.04.2015**
(21) Application number: 13184934.1
(22) Date of filing: 18.09.2013
(51) Int. Cl.: C07C 327/26

(54) **Process for producing aryl ester of thionocarboxylic acid**
Verfahren zur Herstellung von Arylester aus Thionocarboxylsäure
Procédé de production d'aryl ester d'acide thiono-carboxylique

(30) Priority: 24.09.2012 JP 2012209774
(43) Date of publication of application: 26.03.2014
(73) Proprietor: DAIKIN INDUSTRIES, LTD., Osaka-shi, Osaka 530-8323 (JP)
(72) Inventor: Namikawa, Takashi, Osaka, 566-0044 (JP); Kishikawa, Yousuke, Osaka, 566-0044 (JP); Kishimoto, Masayuki, Osaka, 566-0044 (JP); Adachi, Kenji, Osaka, 566-0044 (JP); Ishihara, Sumi, Osaka, 566-0044 (JP); Tanaka, Asako, Osaka, 566-0044 (JP)
(74) Representative: Hoffmann Eitle

(56) References cited:
- HORST VIOLA ET AL: "Organische Schwefelverbindungen,97. Friedel-Crafts-Reaktionen mit Thiosäurechloriden", CHEMISCHE BERICHTE, vol. 101, no. 10, 1 October 1968 (1968-10-01), pages 3517-3529, XP55084572, ISSN: 0009-2940, DOI: 10.1002/cber.19681011024
- J. R. FALCK ET AL: "Tin-Copper Transmetalation: Cross-Coupling of .alpha.-Heteroatom-Substituted Alkyltributylstannanes with Organohalides", JOURNAL OF THE AMERICAN CHEMICAL SOCIETY, vol. 117, no. 22, 1 June 1995 (1995-06-01) , pages 5973-5982, XP55084573, ISSN: 0002-7863, DOI: 10.1021/ja00127a010
- J. FALCK ET AL: "Cu 2 S Mediated Cross-Coupling of [alpha]-Stannylepoxides", SYNLETT, vol. 1997, no. Sup. I, 1 June 1997 (1997-06-01), pages 481-482, XP55084571, ISSN: 0936-5214, DOI: 10.1055/s-1997-6123
- BELOSLUDTSEV Y Y ET AL: "C-Glycosides: Pd/Cu co-catalyzed thiocarboxylation of stannyl glucopyranosides", TETRAHEDRON LETTERS, PERGAMON, vol. 36, no. 33, 14 August 1995 (1995-08-14), pages 5881-5882, XP027242224, ISSN: 0040-4039 [retrieved on 1995-08-14]
- KLAUS HARTKE ET AL: "[alpha],[beta]-acetylenic dithio and thiono esters", TETRAHEDRON LETTERS, vol. 30, no. 9, 1 January 1989 (1989-01-01), pages 1073-1076, XP55084574, ISSN: 0040-4039, DOI: 10.1016/S0040-4039(01)80363-4

## Description

### Technical Field

The present invention relates to a process for producing an aryl ester of aryl-containing thionocarboxylic acid that is useful as a liquid crystal material, etc.

### Background Art

The aryl ester of arylthionocarboxylic acid represented by the formula R¹C=(S)OR², wherein R¹ and R² independently represent an optionally substituted aryl group, is a compound that is useful as a liquid crystal material, etc.

Various methods are known as methods for producing thionocarboxylic acid esters having this structure. Examples of such methods include a method comprising reacting a chlorothionoformic acid ester with an aromatic compound (Non-patent Literature (NPL) 1); a method comprising treating a carbanion with a dithiocarbonic acid ester or a thionocarbonic acid ester (Non-patent Literature (NPL) 2); a method comprising reacting an orthoester with hydrogen sulfide (Non-patent Literature (NPL) 3); a method comprising reacting a carboxylic acid ester with phosphorus pentasulfide or Lawesson's reagent (Non-patent Literature (NPL) 4); a method comprising reacting a thionocarboxylic acid chloride with an alcohol or phenol (Non-patent Literature (NPL) 5); a method comprising reacting a nitrile with an alcohol and then reacting the resulting product with hydrogen sulfide (Non-patent Literature (NPL) 6); a method comprising treating a methylated aromatic compound with sulfur and an alcohol (Non-patent Literature (NPL) 7); and a method comprising reacting a thioacyl disulfide with an alcoholate (Non-patent Literature (NPL) 8).

However, among these methods, the methods disclosed in NPL 1, 2, 4, 7, etc., are low-yield; in particular, the method disclosed in NPL 4 requires a high temperature of 100°C or more for the reaction, and also has a drawback of generating a large amount of by-products; therefore, these methods cannot be regarded as efficient methods.

The methods disclosed in NPL 3 and 6 use hydrogen sulfide, which is so toxic that its handling requires specialized equipment; therefore, these methods are unsuitable as industrial processes for production. Further, the method disclosed in NPL 5 cannot be regarded as a comprehensively superior method due to a low synthesis yield of the starting thionocarboxylic acid chloride. The method disclosed in NPL 8 comprises a large number of steps, including synthesizing a thioacyl disulfide from a dithiocarboxylic acid derivative and then reacting the thioacyl disulfide with an alcoholate; therefore, this method cannot be regarded as a convenient method.

### Citation List

### Non-patent Literature

- NPL 1:: H. Viola, et al., Chem. Ber., 101, 3517 (1968)
- NPL 2:: Liebigs Ann. Chem., 1973, 1637
- NPL 3:: A. Ohno et al., Tetrahedron Lett., 1968, 2083
- NPL 4:: Synthesis, 1973, 149; Bull. Chem. Soc. Belg., 87, 293 (1987)
- NPL 5:: S. Scheithauer et al., Chem. Ber., 98, 838 (1965)
- NPL 6:: Liebigs Ann. Chem., 1974, 671
- NPL 7:: Z. Chem., 6, 108 (1966)
- NPL 8:: K.A. Latif et al., Tetrahedron, 26, 4247 (1970)

### Summary of Invention

### Technical Problem

The present invention was made in view of the above-mentioned problems in the prior art. A primary object of the invention is to provide a relatively convenient and industrially advantageous process for producing, in a high yield, an aryl ester of arylthionocarboxylic acid, which is useful as a liquid crystal material, etc.

### Solution to Problem

To achieve the above object, the present inventors conducted extensive research. As a result, the inventors found that when a halogenated thiocarbonyl compound is used as a starting material and reacted with a specific arylated metal compound, the desired aryl ester of arylthionocarboxylic acid can be produced in a high yield under mild reaction conditions using relatively safe starting materials. The present invention has been accomplished based on this finding.

More specifically, the present invention provides the following processes for producing an aryl ester of arylthionocarboxylic acid.
Item 1. A process for producing an aryl ester of arylthionocarboxylic acid represented by Formula (3): 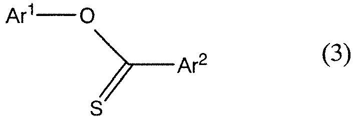
   wherein Ar¹ and Ar² independently represent an optionally substituted aryl group;
   the process comprising reacting a halogenated thiocarbonyl compound represented by Formula (1): 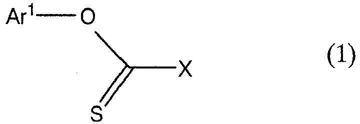 wherein Ar¹ is as defined above and X is halogen, with an arylated metal compound represented by Formula (2): Ar²-M, wherein Ar² is as defined above and M is an alkali metal or monovalent copper.
Item 2. The process according to Item 1, wherein the reaction is performed in an aprotic organic solvent.
Item 3. The process according to Item 1 or 2, wherein the arylated metal compound represented by Formula (2): Ar²-M, wherein Ar² is an optionally substituted aryl group and M is an alkali metal or monovalent copper, is an arylated alkali metal compound represented by Formula (2'): Ar²-M¹, wherein Ar² is as defined above and M¹ is an alkali metal, the arylated alkali metal compound being obtained by reacting an aryl compound represented by Formula (4): Ar²-Y, wherein Ar² is as defined above and Y is hydrogen or halogen other than F, with an organic alkali metal compound represented by Formula (5): R-M¹, wherein R is alkyl or phenyl and M¹ is as defined above.
Item 4. The process according to Item 1 or 2, wherein the arylated metal compound represented by Formula (2): Ar²-M, wherein Ar² is an optionally substituted aryl group and M is an alkali metal or monovalent copper, is an arylated copper compound represented by Formula (2"): Ar²-Cu, wherein Ar² is as defined above, the arylated copper compound being obtained by producing an arylated alkali metal compound represented by Formula (2'): Ar²-M¹, wherein Ar² is as defined above and M¹ is an alkali metal, by the process described in Item 3, and then reacting the arylated alkali metal compound with a monovalent copper compound.
Item 5. The process according to any one of Items 1 to 4, wherein the halogenated thiocarbonyl compound represented by Formula (1) is reacted with the arylated metal compound represented by Formula (2) in the presence of at least one component selected from the group consisting of trivalent iron compounds and divalent nickel compounds.
Item 6. A process for producing an aryl ester of arylthionocarboxylic acid represented by Formula (3): 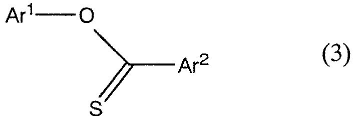
   wherein Ar¹ and Ar² independently represent an optionally substituted aryl group;
   the process comprising the steps of reacting an aryl compound represented by Formula (4): Ar²-Y, wherein Ar² is as defined above and Y is hydrogen or halogen other than F, with an organic alkali metal compound represented by Formula (5): R-M¹, wherein R is alkyl or phenyl and M¹ is an alkali metal; optionally reacting the resulting product with a monovalent copper compound; and then reacting the resulting product with a halogenated thiocarbonyl compound represented by Formula (1): 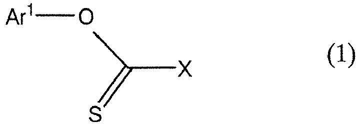 wherein Ar¹ is as defined above and X is halogen.
Item 7. The process according to Item 6, wherein the reaction with the halogenated thiocarbonyl compound represented by Formula (1) is performed in the presence of at least one component selected from the group consisting of trivalent iron compounds and divalent nickel compounds.

The process of the present invention for producing an aryl ester of arylthionocarboxylic acid is explained in more detail below.

The process of the present invention for producing an aryl ester of arylthionocarboxylic acid comprises reacting a halogenated thiocarbonyl compound represented by Formula (1): 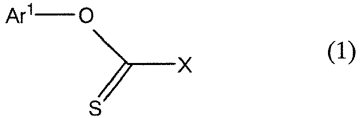 wherein Ar¹ is an optionally substituted aryl group and X is halogen, with an arylated metal compound represented by Formula (2): Ar²-M, wherein Ar² is an optionally substituted aryl group and M is an alkali metal or monovalent copper. This process can produce an aryl ester of arylthionocarboxylic acid represented by Formula (3): 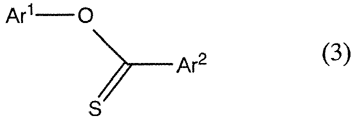 wherein Ar¹ and Ar² are as defined above, in a high yield under relatively mild conditions.

First, the starting compounds used in the production process of the present invention are explained in more detail.

### Starting compounds

### (1) Halogenated thiocarbonyl compound

In the starting halogenated thiocarbonyl compound represented by Formula (1), Ar¹ is an optionally substituted aryl group. Examples of optionally substituted aryl groups include optionally substituted phenyl, optionally substituted naphthyl, and the like.

There is no specific limitation on the substituent on the aryl group, insofar as the substituent is inert to the reaction with an arylated metal compound represented by Formula (2): Ar²-M described below. Specific examples of such substituents include alkyl, fluoroalkyl, alkoxy, fluoroalkoxy, cyano, aryl, halogen, and the like. Among these, examples of alkyl groups include linear or branched lower alkyl groups having about 1 to 5 carbon atoms; and examples of fluoroalkyl groups include linear or branched lower alkyl groups having about 1 to 5 carbon atoms in which hydrogen atoms are partially or completely replaced by fluorine. Examples of alkoxy groups include linear or branched lower alkoxy groups having about 1 to 5 carbon atoms. Examples of fluoroalkoxy groups include linear or branched lower alkoxy groups having about 1 to 5 carbon atoms in which hydrogen atoms are partially or completely replaced by fluorine. Examples of aryl groups as substituents include phenyl, naphthyl, and the like. The substituent aryl groups may further contain a substituent that is inert to the reaction. Examples of halogen atoms include fluorine, chlorine, bromine, and iodine.

In the above Formula (1), the halogen atom represented by X may be, for example, fluorine, chlorine, bromine, or iodine. Chlorine is particularly preferable.

### (2) Arylated Metal compound

In the arylated metal compound represented by Formula (2): Ar²-M, the aryl group represented by Ar² may be, for example, an optionally substituted phenyl group or an optionally substituted naphthyl group. The substituent on the aryl group may be any substituent that is inert to the reaction with a halogenated thiocarbonyl compound represented by Formula (1), and that is also inert to the reaction for producing an arylated metal compound represented by Formula (2): Ar²-M from an aryl compound represented by Formula (4): Ar²-Y described below. Specific examples of such substituents may be the same as the aforementioned examples of substituents on the aryl group represented by Ar¹.

In Formula (2), M is an alkali metal or monovalent copper. Examples of alkali metals include Li, Na, K, and the like.

Among the arylated metal compounds, those wherein M is an alkali metal, i.e., arylated alkali metal compounds represented by Formula (2'): Ar²-M¹, wherein Ar² is as defined above and M¹ is an alkali metal, can be obtained by reacting an aryl compound represented by Formula (4): Ar²-Y, wherein Ar² is as defined above and Y is hydrogen or halogen other than F, with an organic alkali metal compound represented by Formula (5): R-M¹, wherein R is alkyl or phenyl and M¹ is an alkali metal.

In the aryl compound represented by Formula (4): Ar²-Y, the halogen other than F represented by Y may be, for example, chlorine, bromine, or iodine.

In the organic alkali metal compound represented by Formula (5): R-M¹, R is alkyl or phenyl. Examples of alkyl groups include linear or branched alkyl groups having about 1 to 5 carbon atoms.

The amount of the organic alkali metal compound represented by Formula (5): R-M¹ may be about 0.9 to 5.0 mol, and preferably about 0.9 to 1.5 mol, per mol of the aryl compound represented by Formula (4): Ar²-Y.

The reaction of an aryl compound represented by Formula (4): Ar²-Y with an organic alkali metal compound represented by Formula (5): R-M¹ is preferably performed in an aprotic organic solvent that is inert to the reaction. Examples of aprotic organic solvents include tetrahydrofuran, diethyl ether, 2-methyltetrahydrofuran, dibutyl ether, *tert*-butyl methyl ether, cyclopentyl methyl ether, dimethoxyethane, dioxane, benzene, toluene, xylene, pentane, hexane, heptane, and the like. Such aprotic organic solvents may be used as a mixture of two or more, if necessary.

There is no particular limitation on the concentrations of the starting materials in the reaction solvent. For example, the concentration of the aryl compound represented by Formula (4): Ar²-Y may be about 0.1 to 3 mol/L.

The temperature at which the reaction of an aryl compound represented by Formula (4): Ar²-Y with an organic alkali metal compound represented by Formula (5):R-M¹ is performed is preferably not higher than room temperature, and more preferably about -78°C to 2°C.

The pressure during the reaction is not particularly limited, and can typically be performed under atmospheric pressure. The atmosphere during the reaction is not particularly limited. Typically, the reaction is preferably performed in an inert gas atmosphere, such as a nitrogen atmosphere or an argon atmosphere. The reaction time is not particularly limited. Typically, the reaction time may be about 5 minutes to about 10 hours.

According to the above process, an arylated alkali metal compound represented by Formula (2'): Ar²-M¹, wherein Ar² and M¹ are as defined above, can be obtained.

In the present invention, the desired aryl ester of arylthionocarboxylic acid represented by Formula (3): 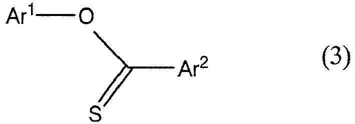 wherein Ar² and M¹ are as defined above, can be obtained by reacting an arylated alkali metal compound represented by Formula (2') obtained by the above method with a halogenated thiocarbonyl compound represented by Formula (1). In particular, the desired aryl ester of arylthionocarboxylic acid represented by Formula (3) can be obtained in an increased yield by reacting an arylated alkali metal compound represented by Formula (2'): Ar²-M¹, wherein Ar² and M¹ are as defined above, with a monovalent copper compound to produce an arylated copper compound represented by Formula (2"): Ar²-Cu wherein Ar² is as defined above, and then reacting the obtained arylated copper compound with a halogenated thiocarbonyl compound represented by Formula (1).

The arylated copper compound represented by Formula (2"): Ar²-Cu can be obtained by reacting an arylated alkali metal compound represented by Formula (2'): Ar²-M¹ with a monovalent copper compound. In this reaction, a copper halide such as copper chloride, copper bromide, or copper iodide; copper cyanide (CuCN), or the like can be used as the monovalent copper compound.

The amount of the monovalent copper compound used may be about 0.1 to 5 mol, and is preferably about 0.7 to 1.2 mol, per mol of the arylated alkali metal compound represented by Formula (2'): Ar²-M¹.

The reaction of the arylated alkali metal compound represented by Formula (2'): Ar²-M¹ with a monovalent copper compound is preferably performed in an aprotic organic solvent. Examples of usable aprotic organic solvents may be the same as the aforementioned examples of solvents that can be used in the reaction of an arylated compound represented by Formula (4): Ar²-Y with an organic alkali metal compound represented by Formula (5): R-M¹.

There is no particular limitation on the concentrations of the starting materials in the solvent. For example, the concentration of the arylated alkali metal compound represented by Formula (2'): Ar²-M¹ may be about 0.1 to 3 mol/L.

The temperature at which the reaction of an arylated alkali metal compound represented by Formula (2'): Ar²-M¹ with a monovalent copper compound is performed is preferably not higher than room temperature, and more preferably about -78°C to room temperature, and even more preferably about -30 to 2°C.

The pressure during the reaction is not particularly limited. The reaction can typically be performed under atmospheric pressure. The atmosphere during the reaction is not particularly limited. Typically, the reaction is preferably performed in an inert gas atmosphere, such as a nitrogen atmosphere or an argon atmosphere. Typically, the reaction time may be about 5 minutes to about 10 hours.

The above process can produce an arylated metal compound represented by Formula (2): Ar²-M wherein M is Cu, i.e., an arylated copper compound represented by Formula (2"): Ar²-Cu.

### Process for producing an aryl ester of arylthionocarboxylic acid

As described above, the desired aryl ester of arylthionocarboxylic acid of the present invention, which is represented by Formula (3): 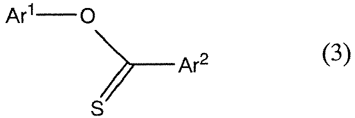 wherein Ar¹ and Ar² independently represent an optionally substituted aryl group, can be obtained by reacting a halogenated thiocarbonyl compound represented by Formula (1): 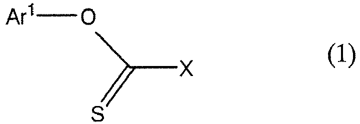 wherein Ar¹ is as defined above and X is halogen, with an arylated metal compound represented by Formula (2): Ar²-M, wherein M is an alkali metal or monovalent copper.

The amount of the arylated metal compound represented by Formula (2): Ar²-M may be about 1 to 1.5 mol, and is preferably about 1 to 1.2 mol, per mol of the halogenated thiocarbonyl compound represented by Formula (1).

The reaction of a halogenated thiocarbonyl compound represented by Formula (1) with an arylated metal compound represented by Formula (2): Ar²-M is preferably performed in an aprotic organic solvent. Examples of usable aprotic organic solvents may be the same as the aforementioned examples of solvents that can be used in the reaction of an aryl compound represented by Formula (4): Ar²-Y with an organic alkali metal compound represented by Formula (5): R-M¹.

There is no particular limitation on the concentrations of the starting materials in the reaction solvent. For example, the concentration of the halogenated thiocarbonyl compound represented by Formula (1) may be about 0.1 to 3 mol/L.

The temperature at which the reaction of a halogenated thiocarbonyl compound with an arylated metal compound is performed is preferably not higher than room temperature, and more preferably about -30 to 2°C.

The pressure during the reaction is not particularly limited. The reaction can typically be performed under atmospheric pressure. The atmosphere during the reaction is not particularly limited. Typically, the reaction is preferably performed in an inert gas atmosphere, such as a nitrogen atmosphere or an argon atmosphere. Typically, the reaction time may be about 5 to 10 hours.

The above process can produce the desired aryl ester of arylthionocarboxylic acid represented by Formula (3): 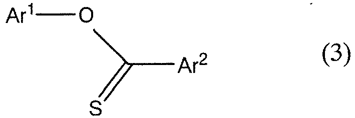 in a high yield. In particular, when using an arylated metal compound represented by Formula (2): Ar²-M wherein M is Cu, i.e., an arylated copper compound represented by Formula (2"): Ar²-Cu, the aryl ester of arylthionocarboxylic acid represented by Formula (3) can be obtained in a particularly high yield.

In the production process of the present invention, the yield of the desired aryl ester of arylthionocarboxylic acid represented by Formula (3) can be increased particularly by reacting a halogenated thiocarbonyl compound represented by Formula (1) with an arylated metal compound represented by Formula (2) in the presence of a catalyst. In particular, when the arylated metal compound represented by Formula (2): Ar²-M is an arylated alkali metal compound represented by Formula (2'): Ar²-M¹, the yield of the aryl ester of arylthionocarboxylic acid represented by Formula (3) can be greatly increased.

Examples of usable catalysts include trivalent iron compounds, divalent nickel compounds, and the like. Such compounds can be used singly, or as a mixture of two or more. Specific examples of trivalent iron compounds include tris(2,4-pentanedionato)iron (III), ferric chloride, and the like. Specific examples of divalent nickel compounds include nickel chloride and the like.

The amount of catalyst is preferably about 0.03 mol or more, per mol of the halogenated thiocarbonyl compound represented by Formula (1). Although there is no particular limitation on the upper limit of the amount of catalyst used, the use of an excess amount of catalyst does not increase the effect, and is disadvantageously costly. Therefore, the amount of catalyst is preferably not more than about 0.5 mol, and more preferably not more than 0.1 mol, per mol of the halogenated thiocarbonyl compound.

The aryl ester of arylthionocarboxylic acid can be purified and collected by a known method, such as silica gel column chromatography or recrystallization.

In the process of the present invention, instead of directly reacting a halogenated thiocarbonyl compound represented by Formula (1): 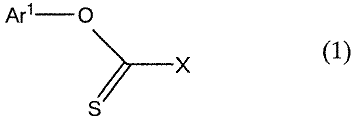 wherein Ar¹ is an optionally substituted aryl group and X is halogen, with an arylated metal compound represented by Formula (2): Ar²-M wherein Ar² is an optionally substituted aryl group and M is a monovalent metal, the following process can also be used to produce the desired aryl ester of arylthionocarboxylic acid represented by Formula (3): 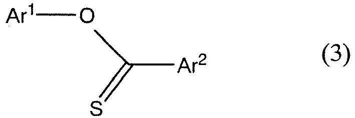 wherein Ar¹ and Ar² are as defined above. That is, an arylated compound represented by Formula (4): Ar²-Y, which is a starting material of an arylated metal compound represented by Formula (2): Ar²-M, is reacted with an organic alkali metal compound represented by Formula (5): R-M¹, the obtained product is optionally reacted with a monovalent copper compound, and then a halogenated thiocarbonyl compound represented by Formula (1): 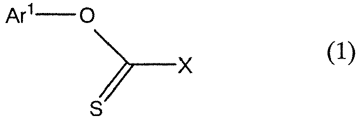 wherein Ar¹ is an optionally substituted aryl group and X is halogen, is added and reacted therewith. This process can produce the desired aryl ester of arylthionocarboxylic acid represented by Formula (3) in a high yield in a continuous manner without separating an arylated alkali metal compound represented by Formula (2'): Ar²-M¹ or an arylated copper compound represented by Formula (2"): Ar²-Cu that is an intermediate product. The reaction conditions in the step of obtaining an arylated alkali metal compound represented by Formula (2'): Ar²-M¹ and in the step of obtaining an arylated copper compound represented by Formula (2") in this continuous process may be the same as the aforementioned conditions in each reaction step.

The aryl ester of arylthionocarboxylic acid obtained by this process can be purified and collected by a known method, such as column chromatography or recrystallization.

### Advantageous Effects of Invention

According to the present invention, the desired aryl ester of arylthionocarboxylic acid can be obtained in a high yield by using relatively inexpensive and highly safe starting materials under mild reaction conditions.

### Description of Embodiments

The present invention is explained in more detail below with reference to Examples.

### Example 1

2,4-Difluorobenzene (2.32 g, 87.7 mmol) and tetrahydrofuran (hereinafter abbreviated as "THF") (31 ml) were added in a nitrogen atmosphere to a 100 ml three-necked flask equipped with a stirring bar and a thermometer. After the mixture was cooled to -50°C, a hexane solution of n-butyl lithium (55 ml, 87.7 mmol) was added dropwise, and the resulting mixture was stirred for 1 hour.

Subsequently, phenyl chlorothionoformate (15.1 g, 87.7 mmol) was added dropwise, and the resulting mixture was stirred for 30 minutes to complete the reaction. This mixture was returned to room temperature, and extracted with toluene/hydrochloric acid. The organic layer was evaporated under reduced pressure. The obtained solid was purified by silica gel column chromatography to give phenyl 2,6-difluorophenylthionobenzoate (7.3 g, 29.0 mmol) as a yellow solid. The yield was 32%.

### Example 2

2,4-Difluorobenzene (2.32 g, 87.7 mmol) and tetrahydrofuran (hereinafter abbreviated as "THF") (31 ml) were added in a nitrogen atmosphere to a 100 ml three-necked flask equipped with a stirring bar and a thermometer. After the mixture was cooled to 2°C, hexane solution of n-butyl lithium (55 ml, 87.7 mmol) was added dropwise. Subsequently, copper chloride (4.34 mg, 43.8 mmol) was added, and the resulting mixture was stirred for 1 hour.

Subsequently, phenyl chlorothionoformate (15.1 g, 87.7 mmol) was added dropwise, and the resulting mixture was stirred for 30 minutes to complete the reaction. This mixture was returned to room temperature, and extracted with toluene/hydrochloric acid. The organic layer was evaporated under reduced pressure.

The obtained solid was purified by recrystallization to give phenyl 2,6-difluorophenylthionobenzoate (20.6 g, 82.3 mmol) as yellow needle-like crystals. The yield was 94%.

### Examples 3 to 18

Using the starting materials shown in Tables 1 and 2, the reaction was performed in the same manner as in Example 2. Tables 1 and 2 show the kinds and yields of the obtained products. The numerical values shown in the copper compound items in the Reagent column of the tables refer to the number of equivalents of each copper compound per equivalent of the lithium compound used.

**Table 1**

| Example | [1] | Starting material | Reagent | | [2] | Solvent | Temp. °C | Product | Yield(%)* |
|---|---|---|---|---|---|---|---|---|---|
| 2 | 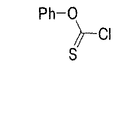 | 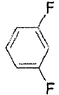 | n-BuLi | CuCl 0.5eq | 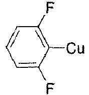 | THF | 2°C | 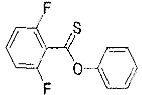 | 94 |
| 3 | 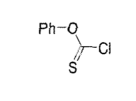 | 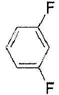 | n-BuLi | CuBr 0.5eq | 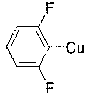 | THF | 2°C | 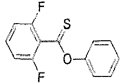 | 92* |
| 4 | 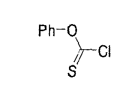 | 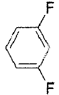 | n-BuLi | CuI 0.5eq | 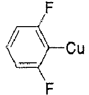 | THF | 2°C | 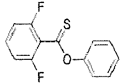 | 95* |
| 5 | 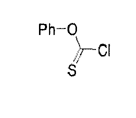 | 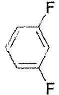 | n-BuLi | CuCN 0.5eq | 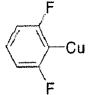 | THF | 2°C | 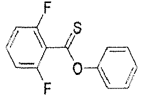 | 92 |
| 6 | 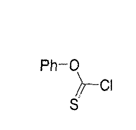 | 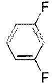 | n-BuLi | CuCl 0.5eq | 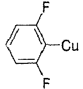 | THF | 2°C | 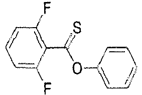 | 88* |
| 7 | 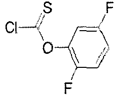 | 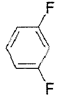 | n-BuLi | CuCl 0.5eq | 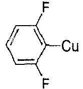 | THF | 2°C | 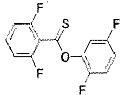 | 90* |
| 8 | 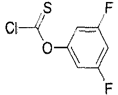 | 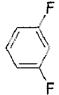 | n-BuLi | CuCl 0.5eq | 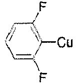 | THF | 2°C | 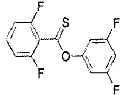 | 92 |
| 9 | 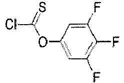 | 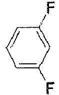 | n-BuLi | CuCl 0.5eq | 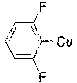 | THF | 2°C | 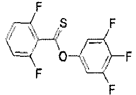 | 95 |

| | | | | | | | | | |
|---|---|---|---|---|---|---|---|---|---|
| * Determined by ¹⁹F NMR | | | | | | | | | |

**Table 2**

| Example | [1] | Starting material | Reagent | | [2] | Solvent | Temp. °C | Product | Yield(%)* |
|---|---|---|---|---|---|---|---|---|---|
| 10 | 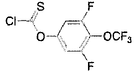 | 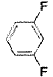 | n-BuLi | CuCl 0.5eq | 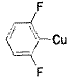 | THF | 2°C | 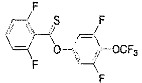 | 76* |
| 11 | 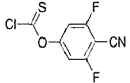 | 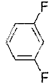 | n-BuLi | CuCl 0.5eq | 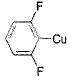 | THF | 2°C | 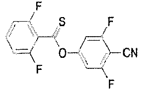 | 77* |
| 12 | 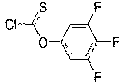 | 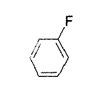 | sec-BuLi | CuCl 0.5eq | 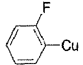 | THF | 2°C | 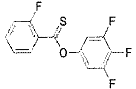 | 76* |
| 13 | 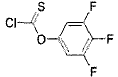 | 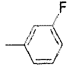 | sec-BuLi | CuCl 0.5eq | 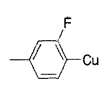 | THF | 2°C | 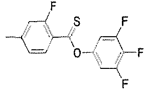 | 88* |
| 14 | 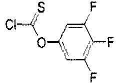 | 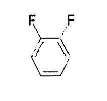 | sec-BuLi | CuCl 0.5eq | 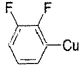 | THF | 2°C | 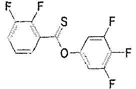 | 92* |
| 15 | 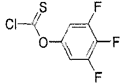 | 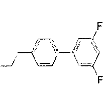 | n-BuLi | CuCl 0.5eq | 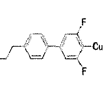 | THF | 2°C | 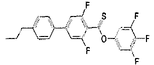 | 95 |
| 16 | 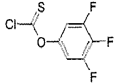 | 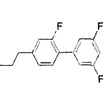 | n-BuLi | CuCl 0.5eq | 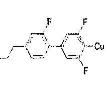 | THF | 2°C | 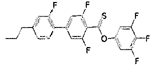 | 94 |
| 17 | 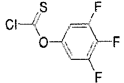 | 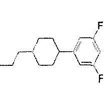 | n-BuLi | CuCl 0.5eq | 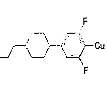 | THF | 2°C | 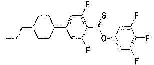 | 88 |
| 18 | 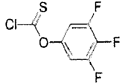 | 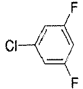 | n-BuLi | CuCl 0.5eq | 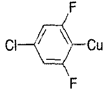 | THF | 2°C | 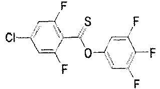 | 92 |
| 19 | 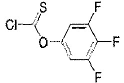 | 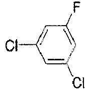 | n-BuLi | CuCl 0.5eq | 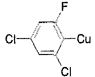 | THF | 2°C | 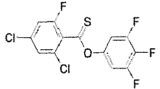 | 88 |
| 20 | 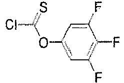 | 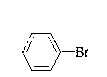 | n-BuLi | CuCl 0.5eq | 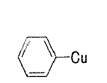 | THF | -78°C | 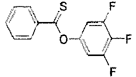 | 67* |

| | | | | | | | | | |
|---|---|---|---|---|---|---|---|---|---|
| * Determined by ¹⁹F NMR | | | | | | | | | |

### Example 21

2,4-Difluorobenzene (2.32 g, 87.7 mmol) and tetrahydrofuran (hereinafter abbreviated as "THF") (31 ml) were added in a nitrogen atmosphere to a 100 ml three-necked flask equipped with a stirring bar and a thermometer. After the mixture was cooled to 2°C, a hexane solution of n-butyl lithium (55 ml, 87.7 mmol) was added dropwise. Subsequently, tris(2,4-pentanedionato)iron (III) (Fe(acac)₃) was added in an amount of 0.1 mol per mol of 2,4-difluorobenzene, and the resulting mixture was stirred for 1 hour.

Subsequently, phenyl chlorothionoformate (15.1 g, 87.7 mmol) was added dropwise, and the resulting mixture was stirred for 30 minutes to complete the reaction. This mixture was returned to room temperature, and extracted with toluene/hydrochloric acid. The organic layer was evaporated under reduced pressure.

The obtained solid was purified by silica gel chromatography to give phenyl 2,6-difluorophenylthionobenzoate as a yellow solid (9.9 g, 39.5 mmol). The yield was 45%.

### Example 22

The same procedure as in Example 21 was repeated, except that instead of using tris(2,4-pentanedionato)iron (III) (Fe(acac)₃), NiCl₂ was used in an amount of 0.1 mol per mol of 2,4-difluorobenzene. Phenyl 2,6-difluorophenylthionobenzoate was obtained as a yellow solid (9.2 g, 35.8 mmol). The yield was 42%.

## Claims

1. A process for producing an aryl ester of arylthionocarboxylic acid represented by Formula (3): 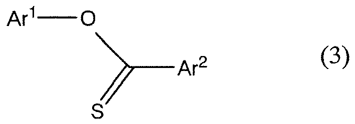
wherein Ar¹ and Ar² independently represent an optionally substituted aryl group;
the process comprising reacting a halogenated thiocarbonyl compound represented by Formula (1): 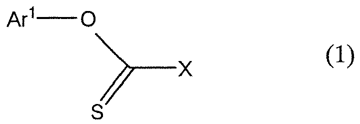 wherein Ar¹ is as defined above and X is halogen, with an arylated metal compound represented by Formula (2): Ar²-M wherein Ar² is as defined above and M is an alkali metal or monovalent copper.

2. The process according to claim 1, wherein the reaction is performed in an aprotic organic solvent.

3. The process according to claim 1 or 2, wherein the arylated metal compound represented by Formula (2): Ar²-M, wherein Ar² is an optionally substituted aryl group and M is an alkali metal or monovalent copper, is an arylated alkali metal compound represented by Formula (2'): Ar²-M¹ wherein Ar² is as defined above and M¹ is an alkali metal,
the arylated alkali metal compound being obtained by reacting an aryl compound represented by Formula (4): Ar²-Y, wherein Ar² is as defined above and Y is hydrogen or halogen other than F, with an organic alkali metal compound represented by Formula (5): R-M¹ wherein R is alkyl or phenyl and M¹ is as defined above.

4. The process according to claim 1 or 2, wherein the arylated metal compound represented by Formula (2): Ar²-M, wherein Ar² is an optionally substituted aryl group and M is an alkali metal or monovalent copper, is an arylated copper compound represented by Formula (2"): Ar²-Cu wherein Ar² is as defined above,
the arylated copper compound being obtained by producing an arylated alkali metal compound represented by Formula (2'): Ar²-M¹, wherein Ar² is as defined above and M¹ is an alkali metal, by the process described in Claim 3, and then reacting the arylated alkali metal compound with a monovalent copper compound.

5. The process according to any one of claims 1 to 4, wherein the halogenated thiocarbonyl compound represented by Formula (1) is reacted with the arylated metal compound represented by Formula (2) in the presence of at least one component selected from the group consisting of trivalent iron compounds and divalent nickel compounds.

6. A process for producing an aryl ester of arylthionocarboxylic acid represented by Formula (3): 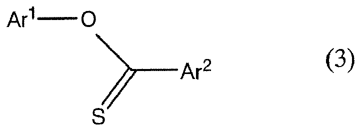
wherein Ar¹ and Ar² independently represent an optionally substituted aryl group;
the process comprising the steps of reacting an aryl compound represented by Formula (4): Ar²-Y, wherein Ar² is as defined above and Y is hydrogen or halogen other than F, with an organic alkali metal compound represented by Formula (5): R-M¹, wherein R is alkyl or phenyl and M¹ is an alkali metal; optionally reacting the resulting product with a monovalent copper compound; and then reacting the resulting product with a halogenated thiocarbonyl compound represented by Formula (1): 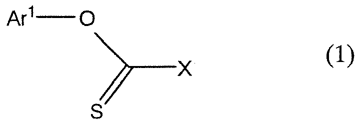 wherein Ar¹ is as defined above and X is halogen.

7. The process according to claim 6, wherein the reaction with the halogenated thiocarbonyl compound represented by Formula (1) is performed in the presence of at least one component selected from the group consisting of trivalent iron compounds and divalent nickel compounds.

## Patentansprüche

1. Verfahren zur Erzeugung eines Arylesters von Arylthionocarbonsäure, dargestellt durch die Formel (3): 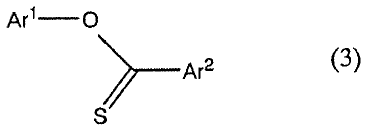
worin Ar¹ und Ar² unabhängig eine wahlweise substituierte Arylgruppe sind;
worin das Verfahren die Reaktion einer halogenierten Thiocarbonylverbindung mit der Formel (1): 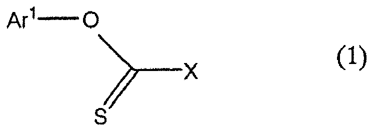 worin Ar¹ wie oben definiert ist und X Halogen ist, mit einer arylierten Metallverbindung mit der Formel (2): Ar²-M, worin Ar² wie oben definiert ist und M ein Alkalimetall oder monovalentes Kupfer ist, umfasst.

2. Verfahren nach Anspruch 1, worin die Reaktion in einem aprotischen organischen Lösungsmittel durchgeführt wird.

3. Verfahren nach Anspruch 1 oder 2, worin die arylierte Metallverbindung, dargestellt durch die Formel (2): Ar²-M, worin Ar² eine wahlweise substituierte Arylgruppe und M ein Alkalimetall oder monovalentes Kupfer ist, eine arylierte Alkalimetallverbindung mit der Formel (2') ist: Ar²-M¹, worin Ar² wie oben definiert ist und M¹ ein Alkalimetall ist,
wobei die arylierte Alkalimetallverbindung erhalten ist durch Reaktion einer Arylverbindung mit der Formel (4): Ar²-Y, worin Ar² wie oben definiert ist und Y Wasserstoff oder ein anderes Halogen als F ist, mit einer organischen Alkalimetallverbindung, dargestellt durch die Formel (5): R-M¹, worin R Alkyl oder Phenyl ist und M wie oben definiert ist.

4. Verfahren nach Anspruch 1 oder 2, worin die arylierte Metallverbindung, dargestellt durch die Formel (2): Ar²-M, worin Ar² eine wahlweise substituierte Arylgruppe und M ein Alkalimetall oder monovalentes Kupfer ist, eine arylierte Kupferverbindung mit der Formel (2"): Ar²-Cu ist, worin Ar² wie oben definiert ist,
worin die arylierte Kupferverbindung erhalten ist durch Erzeugung einer arylierten Alkalimetallverbindung mit der Formel (2'): Ar²-M¹, worin Ar² wie oben definiert ist und M¹ ein Alkalimetall ist, durch das Verfahren, das in Anspruch 3 beschrieben ist, und anschließende Reaktion der arylierten Alkalimetallverbindung mit einer monovalenten Kupferverbindung.

5. Verfahren nach einem der Ansprüche 1 bis 4, worin die halogenierte Thiocarbonylverbindung mit der Formel (1) mit der arylierten Metallverbindung mit der Formel (2) in Gegenwart von zumindest einer Komponente reagiert wird, ausgewählt aus der Gruppe bestehend aus trivalenten Eisenverbindungen und bivalenten Nickelverbindungen.

6. Verfahren zur Erzeugung eines Arylesters von Arylthionocarbonsäure mit der Formel (3): 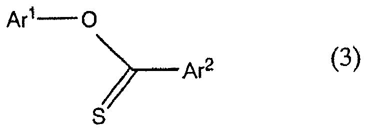
worin Ar¹ und Ar² unabhängig eine wahlweise substituierte Arylgruppe sind;
wobei das Verfahren die Schritte der Reaktion einer Arylverbindung mit der Formel (4): Ar²-Y, worin Ar² wie oben definiert ist und Y Wasserstoff oder ein anderes Halogen ist als F, mit einer organischen Alkalimetallverbindung mit der Formel (5): R-M¹, worin R Alkyl oder Phenyl ist und M¹ ein Alkalimetall ist, wahlweise Reaktion des resultierenden Produktes mit einer monovalenten Kupferverbindung und anschließenden Reaktion des resultierenden Produktes mit einer halogenierten Thiocarbonylverbindung mit der Formel (1) umfasst: 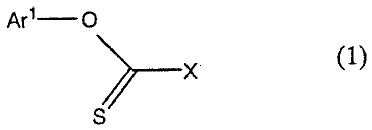 worin Ar¹ wie oben definiert ist und X Halogen ist.

7. Verfahren nach Anspruch 6, worin die Reaktion mit der halogenierten Thiocarbonylverbindung mit der Formel (1) in der Gegenwart von zumindest einer Komponente durchgeführt wird, ausgewählt aus der Gruppe bestehend aus trivalenten Eisenverbindungen und bivalenten Nickelverbindungen.

## Revendications

1. Procédé de production d'un ester arylique d'un acide arylthionocarboxylique représenté par la Formule (3) : 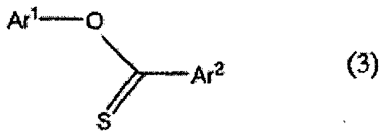
dans laquelle Ar¹ et Ar² représentent indépendamment un groupe aryle facultativement substitué ;
le procédé comprenant la réaction d'un composé thiocarbonyle halogéné représenté par la Formule (1) : 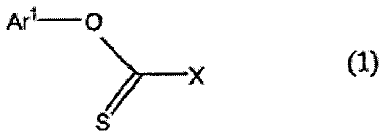 dans laquelle Ar¹ est comme défini ci-dessus et X est un halogène, avec un composé de métal arylé représenté par la Formule (2) : Ar²-M dans laquelle Ar² est comme défini ci-dessus et M est un métal alcalin ou un cuivre monovalent.

2. Procédé selon la revendication 1, dans lequel la réaction est mise en oeuvre dans un solvant organique aprotique.

3. Procédé selon la revendication 1 ou 2, dans lequel le composé de métal arylé représenté par la Formule (2) : Ar²-M, dans laquelle Ar² est un groupe aryle facultativement substitué et M est un métal alcalin ou un cuivre monovalent, est un composé de métal alcalin arylé représenté par la Formule (2') : Ar²-M¹ dans laquelle Ar² est comme défini ci-dessus et M¹ est un métal alcalin,
le composé de métal alcalin arylé étant obtenu par réaction d'un composé aryle représenté par la Formule (4) : Ar²-Y, dans laquelle Ar² est comme défini ci-dessus et Y est un hydrogène ou un halogène autre que F, avec un composé de métal alcalin organique représenté par la Formule (5) : R-M¹ dans laquelle R est un alkyle ou un phényle et M¹ est comme défini ci-dessus.

4. Procédé selon la revendication 1 ou 2, dans lequel le composé de métal arylé représenté par la Formule (2) : Ar²-M, dans laquelle Ar² est un groupe aryle facultativement substitué et M est un métal alcalin ou un cuivre monovalent, est un composé de cuivre arylé représenté par la Formule (2") : Ar²-Cu dans laquelle Ar² est comme défini ci-dessus,
le composé de cuivre arylé étant obtenu par production d'un composé de métal alcalin arylé représenté par la Formule (2') : Ar²-M¹ dans laquelle Ar² est comme défini ci-dessus et M¹ est un métal alcalin, par le procédé décrit dans la revendication 3, et ensuite par réaction du composé de métal alcalin arylé avec un composé de cuivre monovalent.

5. Procédé selon l'une quelconque des revendications 1 à 4, dans lequel le composé thiocarbonyle halogéné représenté par la Formule (1) est fait réagir avec le composé de métal arylé représenté par la Formule (2) en présence d'au moins un composant choisi parmi le groupe consistant en des composés de fer trivalent et des composés de nickel divalent.

6. Procédé de production d'un ester arylique d'un acide arylthionocarboxylique représenté par la Formule (3) : 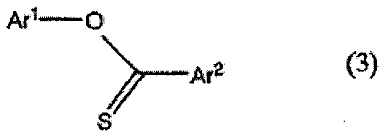
dans laquelle Ar¹ et Ar² représentent indépendamment un groupe aryle facultativement substitué ;
le procédé comprenant les étapes de réaction d'un composé aryle représenté par la Formule (4) : Ar²-Y, dans laquelle Ar² est comme défini ci-dessus et Y est un hydrogène ou un halogène autre que F, avec un composé de métal alcalin organique représenté par la Formule (5) : R-M¹ dans laquelle R est un alkyle ou un phényle et M¹ est un métal alcalin ; de réaction facultative du produit résultant avec un composé de cuivre monovalent ; et ensuite de réaction du produit résultant avec un composé thiocarbonyle halogéné représenté par la Formule (1) : 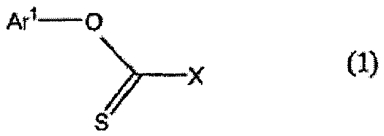 dans laquelle Ar¹ est comme défini ci-dessus et X est un halogène.

7. Procédé selon la revendication 6, dans lequel la réaction avec le composé thiocarbonyle halogéné représenté par la Formule (1) est mise en oeuvre en présence d'au moins un composant choisi parmi le groupe consistant en des composés de fer trivalent et des composés de nickel divalent.
